# EUROPEAN PATENT APPLICATION

(11) **EP 4 586 267 A1**
(43) Date of publication of application: **16.07.2025**
(21) Application number: 25150776.0
(22) Date of filing: 08.01.2025
(51) Int. Cl.: G16H 30/40, G16H 50/30

(54) **PLANNING SYSTEM FOR PREVENTING INTRAOPERATIVE BONE FRACTURE**

(30) Priority: 10.01.2024 US 202463619647 P
(71) Applicant: Zimmer, Inc., Warsaw, IN 46580 (US)
(72) Inventor: FAVRE, Philippe, Zurich (CH); BISCHOFF, Jeffrey E., Holland (US); MAQUER, Ghislain, Zurich (CH); FLEPS, Ingmar, Boston (US)
(74) Representative: Taor, Simon Edward William

(57) **Abstract**

A method to preoperatively assess a risk of bone fracture during or after installing a prosthetic implant into a bone of a patient, the method can include accessing patient medical information. The patient medical information can include at least a medical image of the patient. The medical image can include at least the bone that will receive the prosthetic implant. The method can also include analyzing the medical image to obtain a physical characteristic of the bone. Determining a fracture risk score based on the physical characteristic of the bone. The method can also include transmitting a fracture risk summary to a medical professional. The fracture risk summary can include at least the fracture risk score.

## Description

### CLAIM OF PRIORITY

This application claims the benefit of U.S. Provisional Patent Application Serial No. 63/619,647, filed on January 10, 2024, the benefit of priority of which is claimed hereby, and which is incorporated by reference herein in its entirety.

### TECHNICAL FIELD

Examples described herein generally relate to a medical planning system. More specifically, examples described herein generally relate to a medical planning system for preventing intraoperative bone fractures.

### BACKGROUND

When preparing a bone cavity to receive an implant, such as by rasping and reaming, a surgeon can transmit large forces to the bone. These high forces can negatively affect the bone's resistance to fracture, especially in cases of poor bone quality, thin cortical thickness, unfavorable bone shape, oversized implants, etc. The thickness, density, and shape of the cortical bone directly impact the risk of fracture when preparing the bone for the implant or impacting the implant into the prepared bone. A bone with a thick cortex can better accommodate an implant that fills the canal more and withstands higher impaction forces. In contrast, a bone with a thinner cortex may fracture under similar conditions. Additionally, the morphology of the bone and implant influences fracture risk. For example, long bones can be straight or funnel-shaped. Depending on the shape of the implant, a thin or funnel-shaped cortex could generate high stresses during rasping or implant insertion, increasing fracture risk. Since bone quality and morphology vary between patients, methods to appropriately assess each individual's fracture risk are needed.

### SUMMARY

In examples, a method to preoperatively assess a risk of bone fracture during or after installing a prosthetic implant into a bone of a patient, the method can include accessing patient medical information, the patient medical information including at least a medical image of the patient, the medical image including at least the bone that will receive the prosthetic implant, analyzing the medical image to obtain a physical characteristic of the bone, determining a fracture risk score based on the physical characteristic of the bone, and transmitting a fracture risk summary to a medical professional, the fracture risk summary including at least the fracture risk score.

In examples, at least one non-transitory computer-readable medium, the at least one non-transitory computer-readable medium including instructions that when executed by processing circuitry, cause the processing circuitry to perform operations that can include accessing patient medical information, the patient medical information including at least a medical image of a patient, the medical image including at least a bone that will receive a prosthetic implant, analyze the medical image to obtain a physical characteristic of the bone, determine a fracture risk score based on the physical characteristic of the bone, and transmit a fracture risk summary to a medical professional, the fracture risk summary including at least the fracture risk score.

In examples, a computing apparatus can include a processor. The computing apparatus can also include a memory including instructions that, when executed by the processor, configure the computing apparatus to access patient medical information, the patient medical information including at least a medical image of a patient, the medical image including at least a bone that will receive a prosthetic implant, analyze the medical image to obtain a physical characteristic of the bone, determine a fracture risk score based on the physical characteristic of the bone, and transmit a fracture risk summary to a medical professional, the fracture risk summary including at least the fracture risk score.

In examples, a method for preoperatively assessing a risk of periprosthetic bone fracture, the method can include accessing a medical image of a bone of a patient, determining, from the medical image, one or more cortical bone attributes of the bone, inputting the one or more cortical bone attributes into a machine-learned model trained to predict risk of periprosthetic bone fracture from cortical bone attributes, and generating from the machine-learned model a risk assessment of periprosthetic bone fracture for the patient.

### BRIEF DESCRIPTION OF THE DRAWINGS

Various examples are illustrated in the figures of the accompanying drawings. Such examples are demonstrative and not intended to be exhaustive or exclusive examples of the present subject matter.
FIG. 1 illustrates a schematic diagram of an example of a medical planning system.
FIG. 2 illustrates a flowchart of an example method for a medical planning system.
FIG. 3 illustrates a schematic diagram of an example of a process flowchart for an example medical planning system.
FIG. 4 illustrates a flowchart of an example method for a medical planning system.
FIG. 5 is a block diagram illustrating an example of a machine upon which one or more examples may be implemented.
FIG. 6 illustrates a machine-learning pipeline, according to some examples.
FIG. 7 illustrates training and use of a machine-learning program, according to some examples.

### DETAILED DESCRIPTION

Surgeons can subjectively assess a patient's risk of intraoperative bone fracture during the medical procedure. This assessment can be based on factors evident in medical images, such as bone physical characteristics of the bone, as well as patient demographics and medical conditions. However, this approach is highly subjective and unreliable. Some major drawbacks of this method include that it is a highly subjective approach, which depends on surgeon experience, and because it is subjective, the surgeon can make an error while accessing the risk of bone fracture during the medical procedure. Moreover, this analysis by the surgeon occurs intraoperatively, when the patient is under anesthesia, which leaves little room to adapt the surgical plan if a fracture risk is found to be too high during reaming or implant insertion.

This disclosure relates to a method of objectively, quantitatively, and preoperatively assessing bone fracture risk. The method can include determining bone characteristics, such as cortical thickness (the dense outer layer of bone), density, or bone shape from medical images. This system can also utilize patient information like age, gender, and other traits that could further refine fracture prediction.

The physical bone characteristics can be obtained from preoperative X-rays (imaging that produce images of the structures inside the body), CT scans (Computerized Tomography scans that use computers and rotating X-ray machines to create cross-sectional images of the body), MRIs (Magnetic Resonance Imaging scans that use magnetic fields and radio waves to produce detailed images of the organs and tissues within the body) or other medical images. Patient information can include age, gender, osteoporosis, lifestyle characteristics (e.g., smoking or drinking habits), or other attributes that help predict fracture likelihood. The fracture risk assessment can be provided to the surgeon preoperatively.

When the fracture risk assessment has a higher risk of bone fracture, the assessment can suggest that the surgeon uses lower impact hits when inserting the rasp or implant, opt for a smaller stem size, a less cortical filling stem, a different implant, a different instrumentation system (compaction broach vs rasp) or a different implant shape. This pre-operative information can also be used to set the maximum impaction energy when using robotics surgery, or impactors that control the energy during insertion. The inventors of the present disclosure have found at least the following benefits in using the planning system for preventing intraoperative bone fractures. The system helps prevent intraoperative fractures. The system can help the surgeon know what to expect during the medical procedure, an appropriate implant system or medical procedure can be predicted, the surgical time can be reduced as there is less intraoperative adaption required by the surgeon.

The above discussion is intended to provide an overview of subject matter of the present patent application. It is not intended to provide an exclusive or exhaustive explanation of the invention. The description below is included to provide further information about the present patent application.

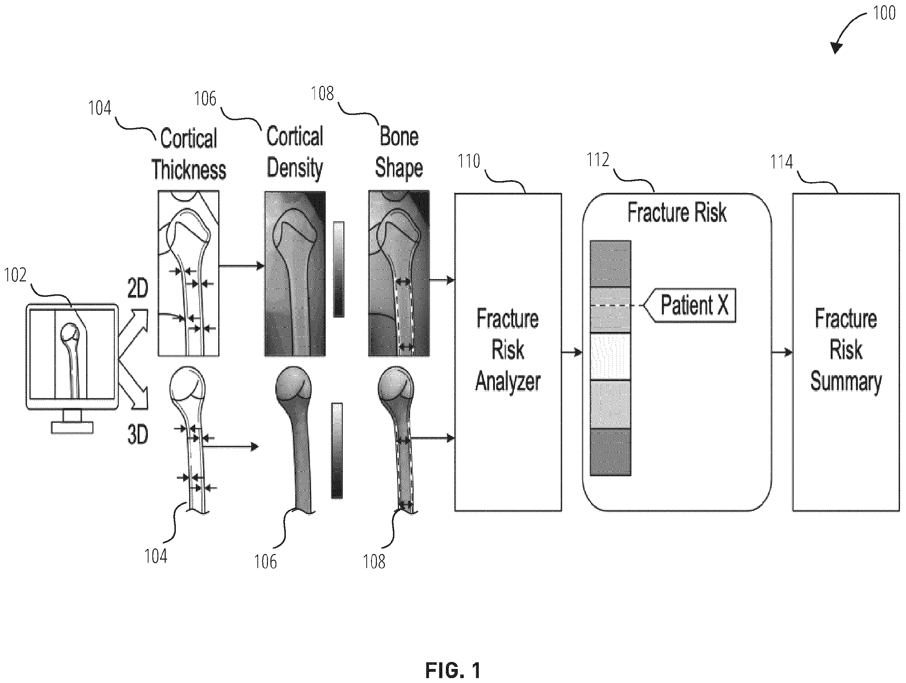

FIG. 1 illustrates a schematic diagram of an example of a medical planning system 100. The medical planning system 100 can be configured to plan a medical procedure. For example, the medical planning system 100 can analyze a planned implant surgery, for a specific patient, and determine a risk of fracture during or after the medical procedure. In examples, the medical planning system 100 can include a patient medical information 102, a cortical thickness analyzer 104, a cortical density analyzer 106, a bone shape analyzer 108, a fracture risk analyzer 110, and any combination of such elements can be used to generate a fracture risk score 112 and a fracture risk summary 114.

The patient medical information 102 can be a computing apparatus, such as a computer, memory, or database, that can store medical images of the patient that were taken pre or intra-operatively. For example, the database can include a complete medical history of the patient, including the images captured in preparation for the medical procedure. Moreover, the patient medical information 102 can be an imaging device, such as an X-ray, computed tomography (CT) scan, magnetic resonance imaging (MRI), or dual X-ray absorptiometry (DEXA), which can be in direct communication with the medical planning system 100.

The cortical thickness analyzer 104 can be a program, algorithm, instructions, module, or the like, configured to analyze the medical image from the patient medical information 102 to determine a cortical thickness of the bone of the patient. For example, the medical planning system 100 or the cortical thickness analyzer 104 can detect the cortical bone thickness using pixel intensity of the medical images or the segmented images. The cortical thickness can be determined by measuring a distance between pixels lying in the inner and outer boundaries of the cortex. In another example, the medical planning system 100 can measure a diameter of the canal (e.g., distance between the cortex of the bone) and determine how the inner canal diameter changes along an axis (e.g., either of the mechanical or anatomical axes). In examples, the cortical bone thickness can be determined using an average thickness along a pre-determined length, or within a portion of the bone, an average thickness along an entire length of a bone, or a minimal thickness of the cortex of the bone along either the entire length or within a targeted region of the bone. In examples, different portions of the bone can have different thresholds for minimum bone thicknesses to determine fracture risk of the bone during preparation of the bone, insertion of the implant, or post-operatively.

The cortical density analyzer 106 can be a program, algorithm, instructions, module, or the like, configured to analyze the medical image from the patient medical information 102 to determine a cortical density of the bone of the patient. In examples, the medical planning system 100 or the cortical density analyzer 106 can be configured to recognize and analyze a contrast or intensity of pixels to determine a cortical density of the bone. This can be done using image processing techniques to determine changes in bone density across a portion or an entire length of the bone, or overall bone density (e.g., maximum, minimum, or average). In examples, different portions of the bone can have different thresholds for minimum bone densities to determine fracture risk of the bone during preparation of the bone, insertion of the implant, or post-operatively.

The bone shape analyzer 108 can be a program, algorithm, instructions, module, or the like, configured to analyze the medical image from the patient medical information 102 to determine a bone shape of the bone of the patient. The medical planning system 100 or the bone shape analyzer 108 can use image processing techniques to determine a bone shape. For example, the bone shape analyzer 108 can use pixel intensity or contrast to determine cortical thickness along an axis of the bone to determine the bone shape. The medical planning system 100 or the bone shape analyzer 108 can also determine changes to the inner canal (e.g., between the cortex of the bone) diameter along a length, or either of the axes (e.g., the mechanical or anatomical axes) to determine bone shape.

In examples, shape modeling techniques can be used by the medical planning system 100 or the cortical thickness analyzer 104, the cortical density analyzer 106, and/or the bone shape analyzer 108 to fit a generic bone shape to specific patient anatomy and determine one or more of the cortical thickness, cortical density, or the bone shape. In examples, the medical planning system 100 or the cortical thickness analyzer 104, the cortical density analyzer 106, and/or the bone shape analyzer 108 can utilize a trained machine learning model to determine or verify any one of the cortical thickness, cortical density, or bone shape.

The fracture risk analyzer 110 can be a program, algorithm, instructions, module, or the like, configured to analyze at least any one of the cortical thickness, cortical density, or bone shape to determine the fracture risk score 112. The fracture risk analyzer 110 can also include other medical information from the patient, such as, age, gender, activity level, comorbidities, lifestyle factors (e.g., smoking status, alcohol consumption, drug use, or the like), or other patient specific health factors.

In examples, the fracture risk analyzer 110 or the medical planning system 100 can utilize the cortical thickness found by the cortical thickness analyzer 104, the cortical density found by the cortical density analyzer 106, the bone shape found by the bone shape analyzer 108, and the patient medical information, to find the fracture risk score by grading each factor individually or in combination. Therefore, the fracture risk can be determined using a sum or mean of each fracture risk score for each parameter.

In examples, the medical planning system 100 or the fracture risk analyzer 110 can analyze the fracture risk for the patient using previous biomechanical testing on cadaveric tissue, which can correlate cortical thickness, cortical density, and bone shape to a risk of fracture during or after the medical procedure. The medical planning system 100 or the fracture risk analyzer 110 can also use computer simulation (such as finite element analysis (FEA)) to predict fracture risks for various bones including various cortical thicknesses, cortical densities, and bone shapes. The simulation can also take into account the implant system, implant sizes, impaction force, or any other physical parameters of the medical procedure to determine different fracture risk scores for all various implant scenarios. The measured cortical thickness, cortical density and bone shape and the planned implant type, planned implant procedure, or other physical parameters of the medical procedure can then be compared to the inputs of the various FEA, to help determine a risk of fracture for the patient.

In other examples, the medical planning system 100 or the fracture risk analyzer 110 can be based on statistical data obtained by databases of bones or in literature. Such data can be expanded using information collected by the medical planning system 100 from previous medical procedures. The bone characteristics and patient medical information can then be compared to the distribution of statistical data to find where the patient falls within the population to statistically determine a fracture risk score.

In examples, the medical planning system 100 or the fracture risk analyzer 110 can utilize a trained machine learning model. The machine learning model can be trained using cadaveric data, simulation data or imaging data from patients who experienced periprosthetic fractures, simulation data or imaging data from patients who did not experience periprosthetic fractures, imaging data and patient medical information, or any combination thereof to better predict the fracture risk scores of the patient. Such machine learning models can determine a fracture risk score with or without specifically finding a cortical thickness, cortical density, or bone shape. Additionally, the trained machine learning model can be used to verify or check any outputs from the medical planning system 100 or the fracture risk analyzer 110 before it is sent in the fracture risk score 112 to the surgeon.

The fracture risk score 112 can be a score of a likelihood of fracture during or after the medical procedure. The fracture risk score 112 can be specific to the procedure and the patient. In examples, the fracture risk score 112 can include a numerical representation, such as a number between 1 and 100 or a number between 1 and 10. In other examples, the fracture risk score 112 can be a color-coded indicia, such as, a green, yellow, orange, or red coloring for a little, low, medium, or high risk of fracture. In examples, the fracture risk score 112 can be an alphanumeric indicia using any combination of letters or numbers to assign a score to the patient. In yet another example, the fracture risk score 112 can be a combination of numerical, alphanumerical, and color-coded indicia.

The fracture risk summary 114 can be configured to provide a summary of the medical procedure and the likelihood of fracture based on all of the personal patient factors (e.g., the patient medical information) and physical characteristics of the bone. The fracture risk summary 114 can include the fracture risk score 112 and all information that goes into generating the fracture risk score 112. The fracture risk summary 114 can also include suggested surgical parameters, such as pressure limits during bone preparation or implant insertion steps, limits on procedures (e.g., drill or reamer speeds or maximum forces, or types of instruments used during bone prep or insertion of the prosthetic implant), or the like. The fracture risk summary 114 can also include information that will aid the patient with recovery, such as physical therapy suggestions based on the health of the bone and prosthetic implant as determined by the medical planning system 100.

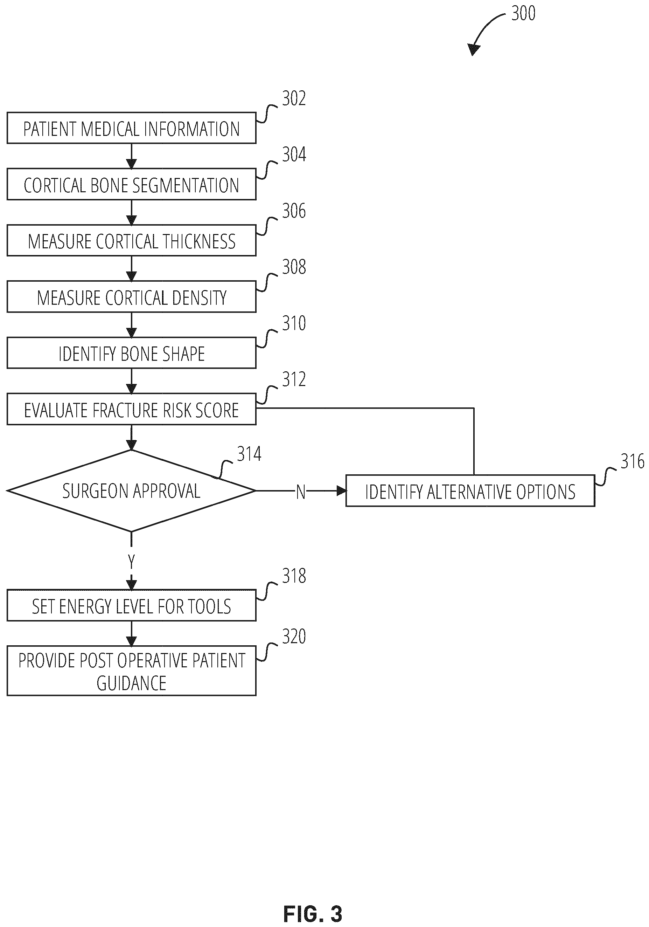

The fracture risk summary 114 can be provided to the surgeon within the preoperative tool for approval. If the risk is low and the surgeon approves the plan, surgery can be performed. If the 114 is rejected, the medical planning system 100 can re-evaluate and generate a new version of the fracture risk summary 114. This process will be discussed with reference to FIG. 3 below.

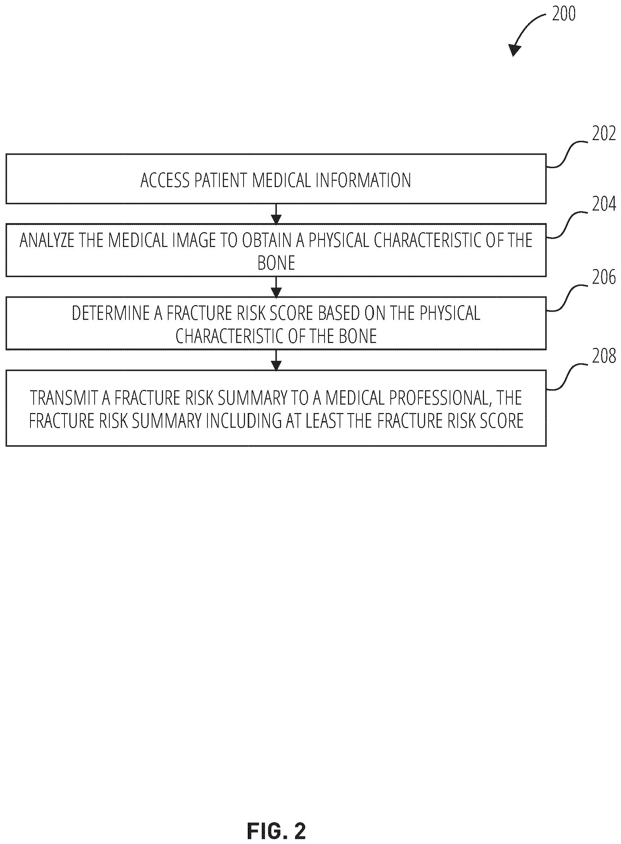

FIG. 2 illustrates a flowchart of an example of a method 200. The method 200 can be for a medical planning system (e.g., the medical planning system 100 from FIG. 1). The method 200 can optionally include any combination of one or more of operations 202-208.

At operation 202, the method 200 can include accessing patient medical information. The patient medical information can include at least a medical image of the patient. The medical image can include at least the bone that will receive the prosthetic implant. In examples, the patient medical information includes a medical history report, and the medical history report can include any one of an age of the patient, a medical diagnosis affecting the bones, or an assigned sex of the patient. The patient medical information can also include at least one of a planned implant type, the planned implant type preoperatively selected by the medical professional; or a planned implant procedure, the planned implant procedure preoperatively selected by the medical professional, the planned implant procedure, which can include one or more of an attachment mechanism to the bone, a maximum force planned for preparation of the bone, or a maximum force planned for impaction of the prosthetic implant within the bone. The patient medical information can also include personal medical information about the patient, such as one or more of age, gender, activity level, or comorbidities. The patient medical information can also include lifestyle information, such as smoking status, alcohol consumption, drug use, exercise habits, or hobbies.

At operation 204, the method 200 can include analyzing the medical image to obtain a physical characteristic of the bone. Analyzing the medical image to obtain the physical characteristics of the bone can include segmenting the bone to obtain a segmented image of the bone from the medical image. Measuring the cortical thickness of the bone using the segmented image of the bone. Measuring the cortical density of the bone using the segmented image of the bone. Analyzing the medical image to obtain the physical characteristic of the bone can also include determining the bone shape of the bone using the segmented image of the bone. The physical characteristics of the bone can be one or more of: a cortical thickness, a cortical density, or a bone shape. In examples, such as when using AI models, analyzing the medical image to obtain a physical characteristic of the bone can include using a trained machine learning model to analyze the image and determine one or more physical characteristics of the bone, such as a cortical thickness, a cortical density, or a bone shape.

At operation 206, the method 200 can include determining a fracture risk score based on the physical characteristics of the bone. In examples, the patient medical information and one or more cortical bone attributes can be input into a machine-learned model to help the medical planning system 100 analyze the patient's fracture risk. Determining the fracture risk score can include analyzing the cortical thickness of the bone, analyzing the cortical density of the bone, analyzing the bone shape of the bone, or analyzing the age of the patient, the medical diagnosis affecting the bones, and the assigned sex.

At operation 208, the method 200 can include transmitting a fracture risk summary to a medical professional. The fracture risk summary can include at least the fracture risk score. The fracture risk summary can include a predicted risk level for the planned implant type, the planned implant procedure, and a surgical plan. The fracture risk summary (e.g., the fracture risk summary 114) can be sent to a medical professional for review either before or during the medical procedure. If the medical professional does not agree with the fracture risk summary, they can reject the plan, and based on receiving a negative signal indicative of the medical professional rejecting the surgical plan, the method 200 can identify one or more alternative implant types or one or more alternative implant procedures for use in the bone of the patient and determine an updated risk summary. The updated risk summary can include an updated predicted risk score for each combination of any of the one or more alternative implant types and any of the one or more alternative implant procedures. The method 200 can then transmit an updated surgical plan based on a combination of one or more alternative implant types and the one or more alternative implant procedures having the lowest risk score to the medical professional for review. If the medical professional rejects the surgical plan again, they can provide guidance as to why it is rejected, and that guidance can help refine future surgical plans generated to solve the medical professional's concerns.

FIG. 3 illustrates a schematic diagram of an example of a process flowchart 300 for an example of the medical planning system 100 (FIG. 1). In examples, the medical planning system 100 can perform one or more operations 302-312 to determine a fracture risk score for a patient, and any one of operations 314-320 to aid the surgeon in preparing for the medical procedure, conducting the medical procedure, and providing post-surgical medical advice to the patient after the medical procedure.

At operation 302, the process flowchart 300 can include accessing patient medical information (e.g., the patient medical information 102 (FIG. 1). In examples, the patient medical information can include an age, sex, height, weight, any bone-related medical diagnosis, or the like. Any information of the patient medical information can be used by the medical planning system 100 to aid in the fracture risk analysis and procedure predictability. Moreover, the patient medical information can include one or more medical images of the bone. As discussed with reference to FIG. 2, the medical images can be either 2D or 3D. In a 2D image, the measure of bone quality, cortical thickness and bone shape can be done at select representative anatomical locations. Alternatively, 2D to 3D methods can be implemented to reconstruct the 3-dimensional shape.

At operation 304, the process flowchart 300 can include segmenting the bone of the patient. In examples, this can include segmenting the cortical regions of the bone to help determine cortical thickness, cortical density, or bone shape. The medical planning system 100 (FIG. 1) can be configured to partition or segment the medical images of the bone to show portions of the bone that pertain to the medical procedure. The segmentations can show a distal portion, proximal portion, or any cross-section (e.g., taken along any of the mechanical axis, anatomical axis, or along any of the coronal plane, transverse plane, or sagittal plane).

At operations 306-310, the process flowchart 300 can optionally include measuring the cortical thickness (with the cortical thickness analyzer 104 (FIG. 1)), measuring the cortical density (with the cortical density analyzer 106 (FIG. 1)), and identifying the bone shape (with the bone shape analyzer 108 (FIG. 1)), respectively. As discussed with reference to FIG. 1, the medical planning system 100 can detect the cortical bone thickness, the cortical density, and determine the bone shape using pixel intensity of the medical images or the segmented images.

At operation 312, the process flowchart 300 can optionally include evaluating the fracture risk score. The fracture risk score (e.g., the fracture risk score 112 (FIG. 1)) can be found using the fracture risk analyzer 110 (FIG. 1). The fracture risk score and a surgical plan can be sent to the surgeon (e.g., as a part of the fracture risk summary 114 (FIG. 1)) for preoperative approval.

If the surgeon does not approve the surgical plan, the process flowchart 300 can include operation 316. At operation 316, the process flowchart 300 can optionally include identifying alternative options. The alternative options can include one or more of one or more alternative implant types, one or more alternative implant procedures, or the like. Each alternative example can be sent back to operation 312 to evaluate a fracture risk score. The updated risk summary can then be sent to the surgeon for approval. In examples, the updated risk summary can include just the new proposed implant type and surgical plan. In other examples, the updated risk summary can include all alternatives found during operation 316 and re-evaluated during operation 312.

Once the surgeon approves the surgical plan, the process flowchart 300 can include operations 318 and 320. At operation 318, the process flowchart 300 can include setting an energy level for tools, including surgical robots and tools used by a surgical robot, used during the medical procedure. For example, operation 318 can include setting an energy level for impaction, reaming, rasping, or any other procedure during the medical procedure. Moreover, at operation 318, the process flowchart 300 can also set threshold values that will generate alarms or alter during the medical procedure if anything is detected to cross a threshold value.

At operation 320, the process flowchart 300 can send the surgeon a summary of the medical procedure, which can include all information collected during the medical procedure and post-operative patient guidance. In examples, the process flowchart 300 can guide the surgeon in providing recommendations to the patient on activities or motions to avoid or for the development of personalized physical therapy plans. This information can be further used to warn the patient when risky activity levels are reached, as measured by means of wearable devices.

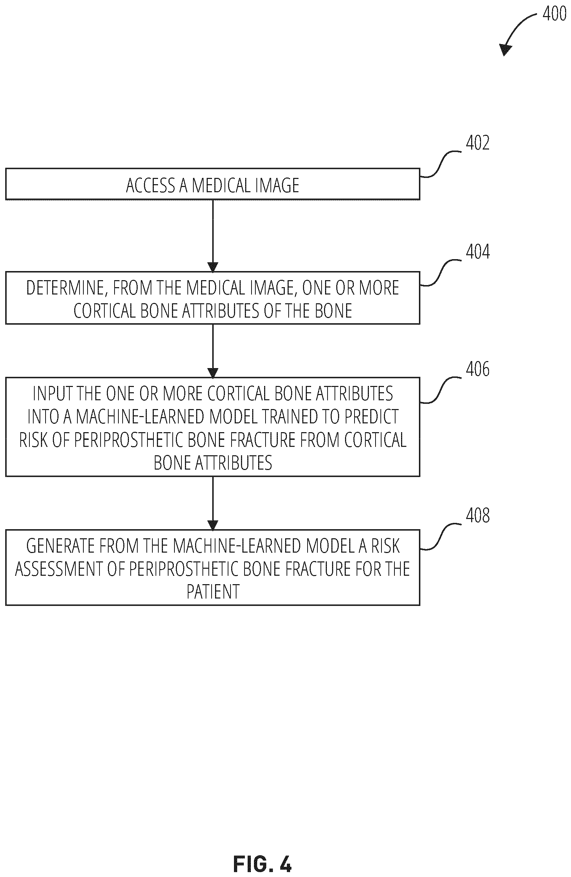

FIG. 4 illustrates a flowchart of an example of a method 400. The method 400 can be for a medical planning system (e.g., the medical planning system 100 (FIG. 1). In examples, the method 400 can include operations 402-408.

At operation 402, the method 400 can include accessing a medical image of a bone of a patient. The medical image can include at least the bone that will receive the prosthetic implant. The medical image can be a 2D, 3D, or CAD model generated from a 2D or 3D model of the bone. In examples, the medical image can be captured at a preoperative appointment, or right before (or during) the surgical procedure.

At operation 404, the method 400 can include determining, from the medical image, one or more cortical bone attributes of the bone. One or more cortical bone attributes can include any one of a cortical bone thickness, a cortical bone density, or a cortical bone shape. In examples, determining one or more cortical bone attributes can include any one of performing image segmentation, shape modeling, or machine learning analysis on the medical image.

At operation 406, the method 400 can include inputting one or more cortical bone attributes into a machine-learned model trained to predict the risk of periprosthetic bone fracture from cortical bone attributes. Patient medical information can also be used during the method 400, the patient medical information can include one or more of age, sex, activity level, comorbidities, smoking status, or alcohol consumption. In examples, the patient medical information can be combined with one or more cortical bone attributes for analysis by the machine-learned model. In examples, the machine-learned model can include a statistical model trained on a database of cortical bone attributes and associated periprosthetic fracture occurrences.

At operation 408, the method 400 can include generating from the machine-learned model a risk assessment of periprosthetic bone fracture for the patient. As discussed herein, the risk assessment and the surgical plan can be provided to the surgeon, who can provide input on the surgical plan and the risk assessment. Upon receiving a rejection, or notes from the surgeon, the method 400 can include re-evaluating options, determining fracture risk scores for each of the alternatives, and transmitting an updated risk assessment to the surgeon for their approval.

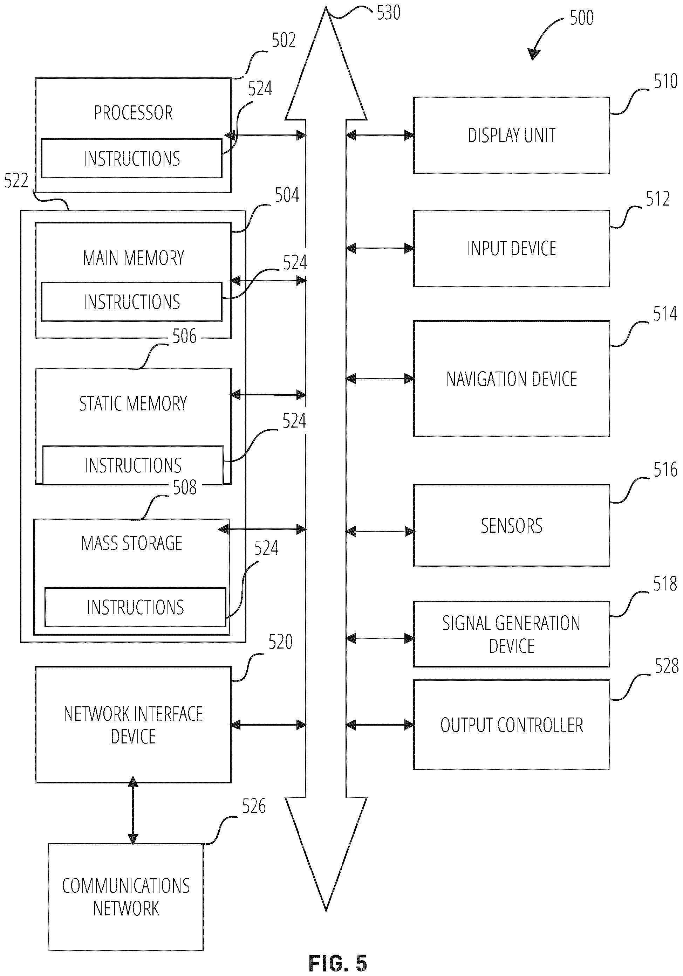

FIG. 5 illustrates a block diagram of an example machine 500 upon which any one or more of the techniques (e.g., methodologies) discussed herein may perform. Examples, as described herein, may include, or may operate by, logic or a number of components, or mechanisms in the machine 500. Circuitry (e.g., processing circuitry) is a collection of circuits implemented in tangible entities of the machine 500 that include hardware (e.g., simple circuits, gates, logic, etc.). Circuitry membership may be flexible over time. Circuitries include members that may, alone or in combination, perform specified operations when operating. In an example, hardware of the circuitry may be immutably designed to carry out a specific operation (e.g., hardwired). In an example, the hardware of the circuitry may include variably connected physical components (e.g., execution units, transistors, simple circuits, etc.) including a machine readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, in an example, the machine-readable medium elements are part of the circuitry or are communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time. Additional examples of these components with respect to the machine 500 follow.

In alternative examples, the machine 500 may operate as a standalone device or may be connected (e.g., networked) to other machines. In a networked deployment, the machine 500 may operate in the capacity of a server machine, a client machine, or both in server-client network environments. In an example, the machine 500 may act as a peer machine in peer-to-peer (P2P) (or other distributed) network environment. The machine 500 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The machine 500 may include a hardware processor 502 (e.g., a central processing unit (CPU), a graphics processing unit (GPU), a hardware processor core, or any combination thereof), a main memory 504, a static memory (e.g., memory or storage for firmware, microcode, a basic-input-output (BIOS), and mass storage 508 (e.g., hard drives, tape drives, flash storage, or other block devices) some or all of which may communicate with each other via an interlink 530 (e.g., bus). The machine 500 may further include a display unit 510, an alphanumeric input device 512 (e.g., a keyboard), and a user interface (UI) navigation device 514 (e.g., a mouse). In an example, the display unit 510, input device 512 and UI navigation device 514 may be a touch screen display. The machine 500 may additionally include a signal generation device 518 (e.g., a speaker), a network interface device 520, and one or more sensors 516, such as a global positioning system (GPS) sensor, compass, accelerometer, or other sensor. The machine 500 may include an output controller 528, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

Registers of the processor 502, the main memory 504, the static memory 506, or the mass storage 508 may be, or include, a machine readable medium 522 on which is stored one or more sets of data structures or instructions 524 (e.g., software) embodying or utilized by any one or more of the techniques or functions described herein. The instructions 524 may also reside, completely or at least partially, within any of registers of the processor 502, the main memory 504, the static memory 506, or the mass storage 508 during execution thereof by the machine 500. In an example, one or any combination of the hardware processor 502, the main memory 504, the static memory 506, or the mass storage 508 may constitute the machine readable media 522. While the machine readable medium 522 is illustrated as a single medium, the term "machine readable medium" may include a single medium or multiple media (e.g., a centralized or distributed database, and/or associated caches and servers) configured to store the one or more instructions 524.

The term "machine readable medium" may include any medium that is capable of storing, encoding, or carrying instructions for execution by the machine 500 and that cause the machine 500 to perform any one or more of the techniques of the present disclosure, or that is capable of storing, encoding or carrying data structures used by or associated with such instructions. Non-limiting machine readable medium examples may include solid-state memories, optical media, magnetic media, and signals (e.g., radio frequency signals, other photon based signals, sound signals, etc.). In an example, a non-transitory machine readable medium comprises a machine readable medium with a plurality of particles having invariant (e.g., rest) mass, and thus are compositions of matter. Accordingly, non-transitory machine-readable media are machine readable media that do not include transitory propagating signals. Specific examples of non-transitory machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

In an example, information stored or otherwise provided on the machine readable medium 522 may be representative of the instructions 524, such as instructions 524 themselves or a format from which the instructions 524 may be derived. This format from which the instructions 524 may be derived may include source code, encoded instructions (e.g., in compressed or encrypted form), packaged instructions (e.g., split into multiple packages), or the like. The information representative of the instructions 524 in the machine readable medium 522 may be processed by processing circuitry into the instructions to implement any of the operations discussed herein. For example, deriving the instructions 524 from the information (e.g., processing by the processing circuitry) may include: compiling (e.g., from source code, object code, etc.), interpreting, loading, organizing (e.g., dynamically or statically linking), encoding, decoding, encrypting, unencrypting, packaging, unpackaging, or otherwise manipulating the information into the instructions 524.

In an example, the derivation of the instructions 524 may include assembly, compilation, or interpretation of the information (e.g., by the processing circuitry) to create the instructions 524 from some intermediate or preprocessed format provided by the machine readable medium 522. The information, when provided in multiple parts, may be combined, unpacked, and modified to create the instructions 524. For example, the information may be in multiple compressed source code packages (or object code, or binary executable code, etc.) on one or several remote servers. The source code packages may be encrypted when in transit over a network and decrypted, uncompressed, assembled (e.g., linked) if necessary, and compiled or interpreted (e.g., into a library, stand-alone executable etc.) at a local machine, and executed by the local machine.

The instructions 524 may be further transmitted or received over a communications network 526 using a transmission medium via the network interface device 520 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), LoRa/LoRaWAN, or satellite communication networks, mobile telephone networks (e.g., cellular networks such as those complying with 3G, 4G LTE/LTE-A, or 5G standards), Plain Old Telephone (POTS) networks, and wireless data networks (e.g., Institute of Electrical and Electronics Engineers (IEEE) 502.11 family of standards known as Wi-Fi^{®}, IEEE 502.15.4 family of standards, peer-to-peer (P2P) networks, among others. In an example, the network interface device 520 may include one or more physical jacks (e.g., Ethernet, coaxial, or phone jacks) or one or more antennas to connect to the communications network 526. In an example, the network interface device 520 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 500, and includes digital or analog communications signals or other intangible medium to facilitate communication of such software. A transmission medium is a machine-readable medium.

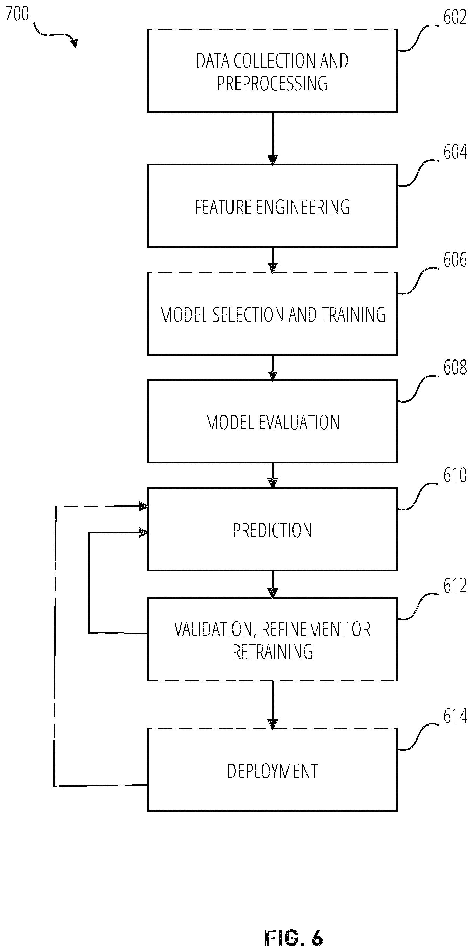

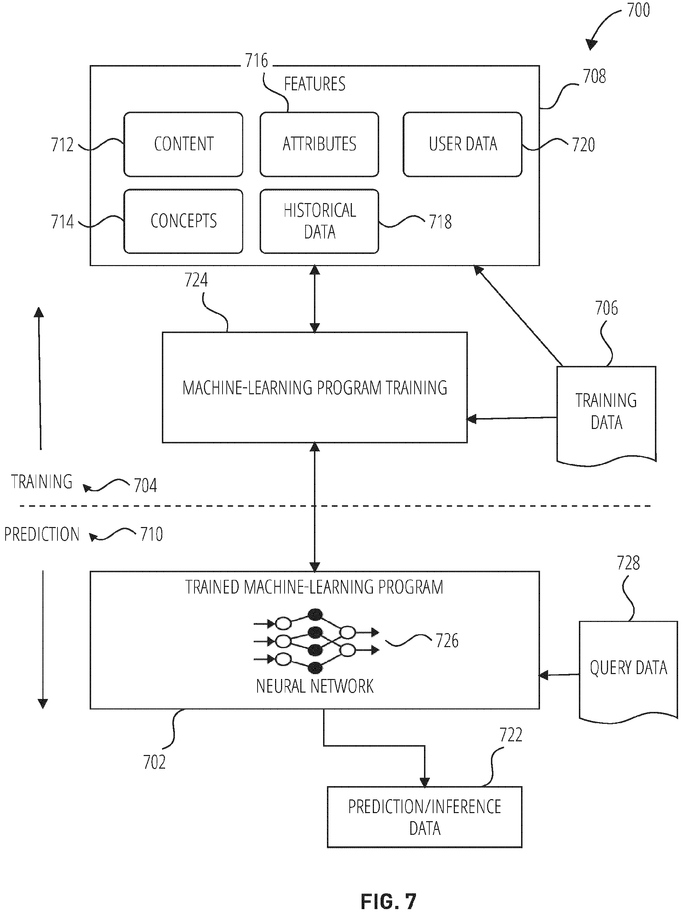

FIGS. 6 and 7 will be discussed together here. FIG. 6 illustrates a machine-learning pipeline 700, according to examples. FIG. 7 is a flowchart depicting a machine-learning pipeline 700, according to examples. The machine-learning pipeline 700 may be used to generate a trained model, for example, the trained machine-learning program 702 of FIG. 7, to perform operations associated with searches and query responses.

### Overview

Broadly, machine learning may involve using computer algorithms to automatically learn patterns and relationships in data, potentially without the need for explicit programming. Machine learning algorithms can be divided into three main categories: supervised learning, unsupervised learning, and reinforcement learning.
- Supervised learning involves training a model using labeled data to predict an output for new, unseen inputs. Examples of supervised learning algorithms include linear regression, decision trees, and neural networks.
- Unsupervised learning involves training a model on unlabeled data to find hidden patterns and relationships in the data. Examples of unsupervised learning algorithms include clustering, principal component analysis, and generative models like autoencoders.
- Reinforcement learning involves training a model to make decisions in a dynamic environment by receiving feedback in the form of rewards or penalties. Examples of reinforcement learning algorithms include Q-learning and policy gradient methods.

Examples of specific machine learning algorithms that may be deployed, according to examples, include logistic regression, which is a type of supervised learning algorithm used for binary classification tasks. Logistic regression models the probability of a binary response variable based on one or more predictor variables. Another example type of machine learning algorithm is Naive Bayes, which is another supervised learning algorithm used for classification tasks. Naive Bayes is based on Bayes' theorem and assumes that the predictor variables are independent of each other. Random Forest is another type of supervised learning algorithm used for classification, regression, and other tasks. Random Forest builds a collection of decision trees and combines their outputs to make predictions. Further examples include neural networks, which consist of interconnected layers of nodes (or neurons) that process information and make predictions based on the input data. Matrix factorization is another type of machine learning algorithm used for recommender systems and other tasks. Matrix factorization decomposes a matrix into two or more matrices to uncover hidden patterns or relationships in the data. Support Vector Machines (SVM) are a type of supervised learning algorithm used for classification, regression, and other tasks. SVM finds a hyperplane that separates the different classes in the data. Other types of machine learning algorithms include decision trees, k-nearest neighbors, clustering algorithms, and deep learning algorithms such as convolutional neural networks (CNN), recurrent neural networks (RNN), and transformer models. The choice of algorithm depends on the nature of the data, the complexity of the problem, and the performance requirements of the application.

The performance of machine learning models is typically evaluated on a separate test set of data that was not used during training to ensure that the model can generalize to new, unseen data.

Although multiple examples of machine learning algorithms are discussed herein, the principles discussed herein can be applied to other machine learning algorithms as well. Deep learning algorithms such as convolutional neural networks, recurrent neural networks, and transformers, as well as more traditional machine learning algorithms like decision trees, random forests, and gradient boosting, may be used in various machine learning applications.

Two example types of problems in machine learning are classification problems and regression problems. Classification problems, also referred to as categorization problems, aim at classifying items into one of several category values (for example, is this object an apple or an orange?). Regression algorithms aim at quantifying some items (for example, by providing a value that is a real number).

### Training phases 704

Generating a trained machine-learning program 702 may include multiple phases that form part of the machine-learning pipeline 700, including for example the following phases illustrated in FIG. 6:
- Data collection and preprocessing 602: This phase may include acquiring and cleaning data to ensure that it is suitable for use in the machine learning model. This phase may also include removing duplicates, handling missing values, and converting data into a suitable format.
- Feature engineering 604: This phase may include selecting and transforming the training data 706 to create features that are useful for predicting the target variable. Feature engineering may include (1) receiving features 708 (e.g., as structured or labeled data in supervised learning) and/or (2) identifying features 708 (e.g., unstructured or unlabeled data for unsupervised learning) in training data 706.
- Model selection and training 606: This phase may include selecting an appropriate machine learning algorithm and training it on the preprocessed data. This phase may further involve splitting the data into training and testing sets, using cross-validation to evaluate the model, and tuning hyperparameters to improve performance.
- Model evaluation 608: This phase may include evaluating the performance of a trained model (e.g., the trained machine-learning program 702) on a separate testing dataset. This phase can help determine if the model is overfitting or underfitting and determine whether the model is suitable for deployment.
- Prediction 610: This phase involves using a trained model (e.g., trained machine-learning program 702) to generate predictions on new, unseen data.
- Validation, refinement, or retraining 612: This phase may include updating a model based on feedback generated from the prediction phase, such as new data or user feedback.
- Deployment 614: This phase may include integrating the trained model (e.g., the trained machine-learning program 702) into a more extensive system or application, such as a web service, mobile app, or IoT device. This phase can involve setting up APIs, building a user interface, and ensuring that the model is scalable and can handle large volumes of data.

FIG. 7 illustrates further details of two example phases, namely a training phase 704 (e.g., part of the model selection and trainings 606) and a prediction phase 710 (part of prediction 610). Prior to the training phase 704, feature engineering 604 is used to identify features 708. This may include identifying informative, discriminating, and independent features for effectively operating the trained machine-learning program 702 in pattern recognition, classification, and regression. In examples, the training data 706 includes labeled data, known for pre-identified features 708 and one or more outcomes. Each of the features 708 may be a variable or attribute, such as an individual measurable property of a process, article, system, or phenomenon represented by a data set (e.g., the training data 706). Features 708 may also be of different types, such as numeric features, strings, and graphs, and may include one or more of content 712, concepts 714, attributes 716, historical data 718, and/or user data 720, merely for example.

In training phase 704, the machine-learning pipeline 700 uses the training data 706 to find correlations among the features 708 that affect a predicted outcome or prediction/inference data 722.

With the training data 706 and the identified features 708, the trained machine-learning program 702 is trained during the training phase 704 during machine-learning program training 724. The machine-learning program training 724 appraises values of the features 708 as they correlate to the training data 706. The result of the training is the trained machine-learning program 702 (e.g., a trained or learned model).

Further, the training phase 704 may involve machine learning, in which the training data 706 is structured (e.g., labeled during preprocessing operations). The trained machine-learning program 702 implements a neural network 726 capable of performing, for example, classification and clustering operations. In other examples, the training phase 704 may involve deep learning, in which the training data 706 is unstructured, and the trained machine-learning program 702 implements a deep neural network 726 that can perform both feature extraction and classification/clustering operations.

In examples, a neural network 226 may be generated during the training phase 704 and implemented within the trained machine-learning program 702. The neural network 726 includes a hierarchical (e.g., layered) organization of neurons, with each layer consisting of multiple neurons or nodes. Neurons in the input layer receive the input data, while neurons in the output layer produce the final output of the network. Between the input and output layers, there may be one or more hidden layers, each consisting of multiple neurons.

Each neuron in the neural network 726 operationally computes a function, such as an activation function, which takes as input the weighted sum of the outputs of the neurons in the previous layer, as well as a bias term. The output of this function is then passed as input to the neurons in the next layer. If the output of the activation function exceeds a certain threshold, an output is communicated from that neuron (e.g., transmitting neuron) to a connected neuron (e.g., receiving neuron) in successive layers. The connections between neurons have associated weights, which define the influence of the input from a transmitting neuron to a receiving neuron. During the training phase, these weights are adjusted by the learning algorithm to optimize the performance of the network. Different types of neural networks may use different activation functions and learning algorithms, affecting their performance on different tasks. The layered organization of neurons and the use of activation functions and weights enable neural networks to model complex relationships between inputs and outputs and to generalize to new inputs that were not seen during training.

In some examples, the neural network 726 may also be one of several different types of neural networks, such as a single-layer feed-forward network, a Multilayer Perceptron (MLP), an Artificial Neural Network (ANN), a Recurrent Neural Network (RNN), a Long Short-Term Memory Network (LSTM), a Bidirectional Neural Network, a symmetrically connected neural network, a Deep Belief Network (DBN), a Convolutional Neural Network (CNN), a Generative Adversarial Network (GAN), an Autoencoder Neural Network (AE), a Restricted Boltzmann Machine (RBM), a Hopfield Network, a Self-Organizing Map (SOM), a Radial Basis Function Network (RBFN), a Spiking Neural Network (SNN), a Liquid State Machine (LSM), an Echo State Network (ESN), a Neural Turing Machine (NTM), or a Transformer Network, merely for example.

In addition to the training phase 704, a validation phase may be performed on a separate dataset known as the validation dataset. The validation dataset is used to tune the hyperparameters of a model, such as the learning rate and the regularization parameter. The hyperparameters are adjusted to improve the model's performance on the validation dataset.

Once a model is fully trained and validated, in a testing phase, the model may be tested on a new dataset. The testing dataset is used to evaluate the model's performance and ensure that the model has not overfitted the training data.

In prediction phase 710, the trained machine-learning program 702 uses features 708 for analyzing query data 728 to generate inferences, outcomes, or predictions, as examples of prediction/inference data 722. For example, during prediction phase 710, the trained machine-learning program 702 generates an output. Query data 728 is provided as an input to the trained machine-learning program 702, and the trained machine-learning program 702 generates the prediction/inference data 722 as output, responsive to receipt of the query data 728.

In examples, the trained machine-learning program 702 may be a generative AI model. Generative AI is a term that may refer to any type of artificial intelligence that can create new content from training data 706. For example, generative AI can produce text, images, video, audio, code, or synthetic data similar to the original data but not identical.

Some of the techniques that may be used in generative AI are:
- Convolutional Neural Networks (CNNs): CNNs may be used for image recognition and computer vision tasks. CNNs may, for example, be designed to extract features from images by using filters or kernels that scan the input image and highlight important patterns.
- Recurrent Neural Networks (RNNs): RNNs may be used for processing sequential data, such as speech, text, and time series data, for example. RNNs employ feedback loops that allow them to capture temporal dependencies and remember past inputs.
- Generative adversarial networks (GANs): GNNs may include two neural networks: a generator and a discriminator. The generator network attempts to create realistic content that can "fool" the discriminator network, while the discriminator network attempts to distinguish between real and fake content. The generator and discriminator networks compete with each other and improve over time.

- Variational autoencoders (VAEs): VAEs may encode input data into a latent space (e.g., a compressed representation) and then decode it back into output data. The latent space can be manipulated to generate new variations of the output data. VAEs may use self-attention mechanisms to process input data, allowing them to handle long text sequences and capture complex dependencies.
- Transformer models: Transformer models may use attention mechanisms to learn the relationships between different parts of input data (such as words or pixels) and generate output data based on these relationships. Transformer models can handle sequential data, such as text or speech, as well as non-sequential data, such as images or code.

In generative AI examples, the output prediction/inference data 222 can include predictions, translations, summaries, or media content.

The following, non-limiting examples, detail certain aspects of the present subject matter to solve the challenges and provide the benefits discussed herein, among others.

Example 1 is a method to preoperatively assess a risk of bone fracture during or after installing a prosthetic implant into a bone of a patient, the method comprising: accessing patient medical information, the patient medical information including at least a medical image of the patient, the medical image including at least the bone that will receive the prosthetic implant; analyzing the medical image to obtain a physical characteristic of the bone; determining a fracture risk score based on the physical characteristic of the bone; and transmitting a fracture risk summary to a medical professional, the fracture risk summary including at least the fracture risk score.

In Example 2, the subject matter of Example 1 optionally includes wherein the physical characteristic of the bone can be one or more of: a cortical thickness; a cortical density; or a bone shape.

In Example 3, the subject matter of Example 2 optionally includes wherein analyzing the medical image to obtain the physical characteristic of the bone comprises: segmenting the bone to obtain a segmented image of the bone from the medical image; measuring the cortical thickness of the bone using the segmented image of the bone; measuring the cortical density of the bone using the segmented image of the bone; and determining the bone shape of the bone using the segmented image of the bone.

In Example 4, the subject matter of Example 3 optionally includes wherein the patient medical information includes a medical history report, and wherein the medical history report comprises any one of an age of the patient, a medical diagnosis affecting the bones, or an assigned sex of the patient.

In Example 5, the subject matter of Example 4 optionally includes wherein determining a fracture risk comprises: analyzing the cortical thickness of the bone; analyzing the cortical density of the bone; analyzing the bone shape of the bone; or analyzing the age of the patient, the medical diagnosis affecting the bones, and the assigned sex.

In Example 6, the subject matter of any one or more of Examples 4-5 optionally include wherein the patient medical information comprises at least one of: a planned implant type, the planned implant type preoperatively selected by the medical professional; or a planned implant procedure, the planned implant procedure preoperatively selected by the medical professional, the planned implant procedure includes one or more of an attachment mechanism to the bone, a maximum force planned for preparation of the bone, or a maximum force planned for impaction of the prosthetic implant within the bone.

In Example 7, the subject matter of Example 6 optionally includes wherein the fracture risk summary includes a predicted risk level for the planned implant type and the planned implant procedure, and wherein the fracture risk summary includes a surgical plan.

In Example 8, the subject matter of Example 7 optionally includes wherein upon receiving a negative signal indicative of the medical professional rejecting the surgical plan, the method comprises: identifying one or more alternative implant types or one or more alternative implant procedures for use in the bone of the patient; determining an updated risk summary, the updated risk summary including an updated predicted risk score for each combination of any of the one or more alternative implant types and any of the one or more alternative implant procedures; or transmitting an updated surgical plan based on a combination of the one or more alternative implant types and the one or more alternative implant procedures having a lowest risk score.

Example 9 is at least one non-transitory computer-readable medium, the at least one non-transitory computer-readable medium including instructions that when executed by processing circuitry, cause the processing circuitry to perform operations comprising: access patient medical information, the patient medical information including at least a medical image of a patient, the medical image including at least a bone that will receive a prosthetic implant; analyze the medical image to obtain a physical characteristic of the bone; determine a fracture risk score based on the physical characteristic of the bone; and transmit a fracture risk summary to a medical professional, the fracture risk summary including at least the fracture risk score.

In Example 10, the subject matter of Example 9 optionally includes wherein the physical characteristic of the bone can be one or more of a cortical thickness, a cortical density, or a bone shape.

In Example 11, the subject matter of Example 10 optionally includes wherein analyzing the medical image to obtain the physical characteristic of the bone comprises any one of: segment the bone to obtain a segmented image of the bone from the medical image; measure the cortical thickness of the bone using the segmented image of the bone; measure the cortical density of the bone using the segmented image of the bone; or determine the bone shape of the bone using the segmented image of the bone.

In Example 12, the subject matter of Example 11 optionally includes wherein the patient medical information includes a medical history report of the patient, and wherein the medical history report of the patient comprises any one of wherein wherein the medical history report comprises any one of an age of the patient, a medical diagnosis affect the bone of the patient, or an assigned sex of the patient.

In Example 13, the subject matter of Example 12 optionally includes wherein determining a fracture risk comprises: analyze the cortical thickness of the bone; analyze the cortical density of the bone; analyze the bone shape of the bone; and analyze the age of the patient, the medical diagnosis affecting the bone of the patient, and the assigned sex of the patient.

In Example 14, the subject matter of any one or more of Examples 9-13 optionally include wherein the patient medical information comprises at least one of: a planned implant type, the planned implant type preoperatively selected by the medical professional; or a planned implant procedure, the planned implant procedure preoperatively selected by the medical professional, the planned implant procedure includes one or more of an attachment mechanism to the bone, a maximum force planned for preparation of the bone, or a maximum force planned for impaction of the prosthetic implant within the bone.

In Example 15, the subject matter of Example 14 optionally includes wherein the fracture risk summary includes a predicted risk level for the planned implant type and the planned implant procedure, and wherein the fracture risk summary includes a surgical plan.

In Example 16, the subject matter of Example 15 optionally includes wherein upon receiving a negative signal indicative of the medical professional reject the surgical plan, the instructions cause the processing circuitry to perform operations comprising: identify one or more alternative implant types for use in the bone of the patient; identify one or more alternative implant procedures for use in the bone of the patient; determine an updated risk summary, the updated risk summary including an updated predicted risk score for each combination of any of the one or more alternative implant types and any of the one or more alternative implant procedures; and transmit an updated surgical plan based on a combination of the one or more alternative implant types and the one or more alternative implant procedures having a lowest risk score.

Example 17 is a computing apparatus comprising: a processor; and a memory including instructions that, when executed by the processor, configure the computing apparatus to: access patient medical information, the patient medical information including at least a medical image of a patient, the medical image including at least a bone that will receive a prosthetic implant; analyze the medical image to obtain a physical characteristic of the bone; determine a fracture risk score based on the physical characteristic of the bone; and transmit a fracture risk summary to a medical professional, the fracture risk summary including at least the fracture risk score.

In Example 18, the subject matter of Example 17 optionally includes wherein the physical characteristic of the bone can be one or more of a cortical thickness, a cortical density, or a bone shape.

In Example 19, the subject matter of Example 18 optionally includes wherein analyzing the medical image to obtain the physical characteristic of the bone comprises the instructions configuring the computing apparatus to perform any one of these operations: segment the bone to obtain a segmented image of the bone from the medical image; measure the cortical thickness of the bone using the segmented image of the bone; measure the cortical density of the bone using the segmented image of the bone; or determine the bone shape of the bone using the segmented image of the bone.

In Example 20, the subject matter of Example 19 optionally includes wherein the patient medical information includes a medical history report of the patient, and wherein the medical history report comprises any one of an age of the patient, a medical diagnosis affect the bone of the patient, or an assigned sex of the patient.

In Example 21, the subject matter of Example 20 optionally includes wherein determining a fracture risk comprises: analyze the cortical thickness of the bone; analyze the cortical density of the bone; analyze the bone shape of the bone; and analyze the age of the patient, the medical diagnosis affecting the bone of the patient, and the assigned sex of the patient.

In Example 22, the subject matter of any one or more of Examples 17-21 optionally include wherein the patient medical information comprises at least one of: a planned implant type, the planned implant type preoperatively selected by the medical professional; or a planned implant procedure, the planned implant procedure preoperatively selected by the medical professional, the planned implant procedure includes one or more of an attachment mechanism to the bone, a maximum force planned for preparation of the bone, or a maximum force planned for impaction of the prosthetic implant within the bone.

In Example 23, the subject matter of Example 22 optionally includes wherein the fracture risk summary includes a predicted risk level for the planned implant type and the planned implant procedure, and wherein the fracture risk summary includes a surgical plan.

In Example 24, the subject matter of Example 23 optionally includes wherein upon receiving a negative signal indicative of the medical professional rejecting the surgical plan, the instructions configure the computing apparatus to perform operations comprising: identify one or more alternative implant types for use in the bone of the patient; identify one or more alternative implant procedures for use in the bone of the patient; determine an updated risk summary, the updated risk summary including an updated predicted risk score for each combination of any of the one or more alternative implant types and any of the one or more alternative implant procedures; and transmit an updated surgical plan based on a combination of the one or more alternative implant types and the one or more alternative implant procedures having a lowest risk score.

Example 25 is a method for preoperatively assessing a risk of periprosthetic bone fracture, the method comprising: accessing a medical image of a bone of a patient; determining, from the medical image, one or more cortical bone attributes of the bone; inputting the one or more cortical bone attributes into a machine-learned model trained to predict risk of periprosthetic bone fracture from cortical bone attributes; and generating from the machine-learned model a risk assessment of periprosthetic bone fracture for the patient.

In Example 26, the subject matter of Example 25 optionally includes wherein the one or more cortical bone attributes of the bone comprises any one of: a cortical bone thickness; a cortical bone density; or a cortical bone shape.

In Example 27, the subject matter of any one or more of Examples 25-26 optionally include wherein determining the one or more cortical bone attributes comprises any one of: performing image segmentation; shape modeling; or machine learning analysis on the medical image.

In Example 28, the subject matter of any one or more of Examples 25-27 optionally include accessing patient information including one or more of: age; gender; activity level; comorbidities; smoking status; or alcohol consumption; and inputting the patient information together with the one or more cortical bone attributes into the machine-learned model.

In Example 29, the subject matter of any one or more of Examples 25-28 optionally include wherein the machine-learned model comprises: a statistical model trained on a database of cortical bone attributes and associated periprosthetic fracture occurrences.

In Example 30, the subject matter of any one or more of Examples 25-29 can optionally include providing the risk assessment to a surgeon and receiving input on a surgical plan based on the risk assessment.

Example 31 is a method, computing apparatus, or system that can optionally include any element from any of Examples 1-30.

The above detailed description includes references to the accompanying drawings, which form a part of the detailed description. The drawings show, by way of illustration, specific examples that may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

All publications, patents, and patent documents referred to in this document are incorporated by reference herein in their entirety, as though individually incorporated by reference. In the event of inconsistent usages between this document and those documents so incorporated by reference, the usage in the incorporated reference(s) should be considered supplementary to that of this document; for irreconcilable inconsistencies, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range (e.g., 1 to 5 includes 1, 1.5, 2, 2.75, 3, 3.90, 4, 4.24, and 5). Similarly, numerical ranges recited herein by endpoints include subranges subsumed within that range (e.g., 1 to 5 includes 1-1.5, 1.5-2, 2-2.75, 2.75-3, 3-3.90, 3.90-4, 4-4.24, 4.24-5, 2-5, 3-5, 1-4, and 2-4). It is also to be understood that all numbers and fractions thereof are presumed to be modified by the term "about."

The above description is intended to be illustrative, and not restrictive. For example, the above-described examples (or one or more aspects thereof) may be used in combination with each other. Other examples may be used, such as by one of ordinary skill in the art upon reviewing the above description. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the examples should be determined with reference to the appended claims, along with the full scope of equivalents to which such claims are entitled.

## Claims

1. A method to preoperatively assess a risk of bone fracture during or after installing a prosthetic implant into a bone of a patient, the method comprising:
accessing patient medical information, the patient medical information including at least a medical image of the patient, the medical image including at least the bone that will receive the prosthetic implant;
analyzing the medical image to obtain a physical characteristic of the bone;
determining a fracture risk score based on the physical characteristic of the bone; and
transmitting a fracture risk summary to a medical professional, the fracture risk summary including at least the fracture risk score.

2. The method of claim 1, wherein the physical characteristic of the bone can be one or more of:
a cortical thickness;
a cortical density; or
a bone shape.

3. The method of claim 2, wherein analyzing the medical image to obtain the physical characteristic of the bone comprises:
segmenting the bone to obtain a segmented image of the bone from the medical image;
measuring the cortical thickness of the bone using the segmented image of the bone;
measuring the cortical density of the bone using the segmented image of the bone; and
determining the bone shape of the bone using the segmented image of the bone.

4. The method of claim 3, wherein the patient medical information includes a medical history report, and wherein the medical history report comprises any one of an age of the patient, a medical diagnosis affecting bone health, or an assigned sex of the patient.

5. The method of claim 4, wherein determining a fracture risk comprises:
analyzing the cortical thickness of the bone;
analyzing the cortical density of the bone;
analyzing the bone shape of the bone; or
analyzing the age of the patient, the medical diagnosis affecting bone health, and the assigned sex.

6. The method of claim 4, wherein the patient medical information comprises at least one of:
a planned implant type, the planned implant type preoperatively selected by the medical professional; or
a planned implant procedure, the planned implant procedure preoperatively selected by the medical professional, the planned implant procedure includes one or more of an attachment mechanism to the bone, a maximum force planned for preparation of the bone, or a maximum force planned for impaction of the prosthetic implant within the bone; and
wherein the fracture risk summary includes a predicted risk level for the planned implant type and the planned implant procedure, and wherein the fracture risk summary includes a surgical plan.

7. The method of claim 6, wherein upon receiving a negative signal indicative of the medical professional rejecting the surgical plan, the method comprises:
identifying one or more alternative implant types or one or more alternative implant procedures for use in the bone of the patient;
determining an updated risk summary, the updated risk summary including an updated predicted risk score for each combination of any of the one or more alternative implant types and any of the one or more alternative implant procedures; or
transmitting an updated surgical plan based on a combination of the one or more alternative implant types and the one or more alternative implant procedures having a lowest risk score.

8. At least one non-transitory computer-readable medium, the at least one non-transitory computer-readable medium including instructions that when executed by processing circuitry, cause the processing circuitry to perform operations comprising:
access patient medical information, the patient medical information including at least a medical image of a patient, the medical image including at least a bone that will receive a prosthetic implant;
analyze the medical image to obtain a physical characteristic of the bone;
determine a fracture risk score based on the physical characteristic of the bone; and
transmit a fracture risk summary to a medical professional, the fracture risk summary including at least the fracture risk score.

9. The at least one non-transitory computer-readable medium of claim 8, wherein the physical characteristic of the bone can be one or more of a cortical thickness, a cortical density, or a bone shape.

10. The at least one non-transitory computer-readable medium of claim 9, wherein analyzing the medical image to obtain the physical characteristic of the bone comprises any one of:
segment the bone to obtain a segmented image of the bone from the medical image;
measure the cortical thickness of the bone using the segmented image of the bone;
measure the cortical density of the bone using the segmented image of the bone; or
determine the bone shape of the bone using the segmented image of the bone.

11. The at least one non-transitory computer-readable medium of claim 10, wherein the patient medical information includes a medical history report of the patient, and wherein the medical history report of the patient comprises any one of an age of the patient, a medical diagnosis affect the bone of the patient, or an assigned sex of the patient.

12. The at least one non-transitory computer-readable medium of claim 11, wherein determining a fracture risk comprises:
analyze the cortical thickness of the bone;
analyze the cortical density of the bone;
analyze the bone shape of the bone; and
analyze the age of the patient, the medical diagnosis affecting the bone of the patient, and the assigned sex of the patient.

13. The at least one non-transitory computer-readable medium of claim 8, wherein the patient medical information comprises at least one of:
a planned implant type, the planned implant type preoperatively selected by the medical professional; or
a planned implant procedure, the planned implant procedure preoperatively selected by the medical professional, the planned implant procedure includes one or more of an attachment mechanism to the bone, a maximum force planned for preparation of the bone, or a maximum force planned for impaction of the prosthetic implant within the bone.

14. The at least one non-transitory computer-readable medium of claim 13, wherein the fracture risk summary includes a predicted risk level for the planned implant type and the planned implant procedure, and wherein the fracture risk summary includes a surgical plan.

15. The at least one non-transitory computer-readable medium of claim 14, wherein upon receiving a negative signal indicative of the medical professional reject the surgical plan, the instructions cause the processing circuitry to perform operations comprising:
identify one or more alternative implant types for use in the bone of the patient;
identify one or more alternative implant procedures for use in the bone of the patient;
determine an updated risk summary, the updated risk summary including an updated predicted risk score for each combination of any of the one or more alternative implant types and any of the one or more alternative implant procedures; and
transmit an updated surgical plan based on a combination of the one or more alternative implant types and the one or more alternative implant procedures having a lowest risk score.
